# EUROPEAN PATENT SPECIFICATION

(11) **EP 3 893 629 B1**
(45) Date of publication and mention of the grant of the patent: **18.11.2026**
(21) Application number: 19842898.9
(22) Date of filing: 29.11.2019
(51) Int. Cl.: A01G 25/16, G01N 27/22, G01N 33/24

(54) **PROBE FOR MEASURING THE PENETRATION TIME OF WATER THROUGH THE SOIL LAYERS AND THE VERTICAL MOISTURE PROFILE OF THE SOIL**
SONDE ZUR MESSUNG DER EINDRINGZEIT VON WASSER DURCH DIE BODENSCHICHTEN UND DES VERTIKALEN FEUCHTIGKEITSPROFILS DES BODENS
SONDE PERMETTANT DE MESURER LE TEMPS DE PÉNÉTRATION DE L'EAU À TRAVERS LES COUCHES DE SOL ET LE PROFIL VERTICAL D'HUMIDITÉ DU SOL

(30) Priority: 30.11.2018 MK 100018
(43) Date of publication of application: 20.10.2021
(73) Proprietor: Indovski, Petar, 1000 Skopje (MK)
(72) Inventor: Indovski, Petar, 1000 Skopje (MK)
(74) Representative: Damjanski, Vanco
(86) International application number: PCT/MK2019/000004
(87) International publication number: WO 2020/111922

(56) References cited:
- EP-A1- 2 883 445
- WO-A1-2006/064266
- US-A1- 2008 129 495
- US-A1- 2017 082 568
- US-A1- 2017 328 854

## Description

### Field of the technique to which the invention relates to

According to the International Patent Classification, the invention " Probe for measuring the penetration time of water through the soil layers and the the vertical moisture profile of the soil" can be classified into the following classes: A01G25, soil moisture control or humidity control devices, i.e. sensors, B05B, dispersion or atomization, generally either the application of liquids or other liquid materials on surfaces, G01, measurement and testing, G01N33, the examination or analysis of materials by determination of their chemical composition or chemical properties, and the class G05, management and regulation.

### State of the art

Soil moisture sensors measure the volumetric water content using certain soil properties such as electrical resistance and dielectric constant. The most commonly used methods for indirectly measuring the volumetric water content in the soil are:
a) Soil electrical conductivity, which is based on the measurement of electrical resistance between two electrodes inserted in the soil, with the resistance decreasing as the soil moisture content increases;
b) Galvanic cell, where the amount of water present is determined on the basis of the voltage generated at the electrodes placed in the soil, with the water acting as an electrolyte and producing electricity;
c) Frequency Domain Reflectometry (FDR), where the dielectric constant of a particular volume element around the sensor is obtained by measuring the operating frequency of an oscillating circuit where there are two types of sensors based on this technology, measurement of capacitance (FD) and electrical impedance (TDT, TDR):
   c1) Time Domain Transmission (TDT), and
   c2) Time Domain Reflectometry (TDR), where the dielectric constant of a particular volume element around the sensor is obtained by measuring the speed of propagation along the transmission line.

Compared to TDR sensors, FD sensors cost less to manufacture and operate with a much shorter measurement cycle. However, due to the complex electric field around the probe, the sensor needs to be calibrated depending on the properties of soil. With the newer generation of sensors, this problem has been lowered by using much higher operating frequencies.

The soil moisture sensors that use soil electrical resistance as method have lowest price, but they are also most inaccurate because of the high influence from the soil environment. Due to the need for direct contact with the soil, metal electrodes must be used which are susceptible to oxidation. These sensors usually measure the average moisture of the soil at a certain depth point at which the probe tip is positioned. They are used for depths in the range of 10 - 25 cm.

The soil moisture sensors which use the FDR capacitive method, measure the average soil moisture. The electrodes of those sensors are made of standard printed circuit board. They are usually used for depths of 10 to 15 cm and have low purchase price.

The sensors using the TDR method generate the most accurate data. These type of probes measure soil moisture in the range of 10 to 15 cm. To measure the soil moisture at multiple depths, multiple probes should be used, by placing each one on desired level in previously excavated hole in the soil.

Multi-level soil moisture sensors, using the FDR method, use sensors made of antirust metal that must ensure good contact with the soil, which is crucial for obtaining accurate measurements. This is achieved by inserting the probe into a precisely made hole in the soil, which must be in strict vertical alignment with the soil surface. To protect the probe from damages, a special procedure is applied which involves pre-compacting in the soil a strong plastic tube with apertures, in which the probe is then inserted. These probes are quite expensive and usually used for scientific research.

A common feature of all of the above mentioned moisture measuring probes is that they do not operate autonomously, that is, additional equipment must be attached, such as a screen reading device, logger, radio transmitter, etc.

Certainly, the state of the technique includes many technical solutions available on the market, but also patented inventions such as patent no. US9.411.070, which relates to a two-way wireless soil moisture measuring device, comprising a set of sensors located one part above the soil surface and the other part located beneath the soil, generating and transmitting soil state data to a controller, with housing for the sensors and the control PCB. The patent US6.975.245 relates to data acquisition control systems and telemetry for the use of real-time irrigation management. Patent US8.035.403 treats wireless sensors that use RF frequency when performing measurements.

The following documents are also known to the state of the art and can be described as the background art.

US 2008/129495 A1 discloses a probe for measurement of the time of penetration of th water through virtual soli layers and the vertical moisture profile.

EP 2 883 445 A1 discloses an extendable wireless soil measurement apparatus including a top head and a plurality of sensors.

US 2017/0328854 A1 discloses an in situ ultra-low power contactless measurement apparatus. WO 2006/064266 discloses a moisture content sensor.

US 2017/082568 A1 discloses a system and method of sensing the amount of moisture in soli. All of the above-mentioned documents differ from the invention in the current application and this will be more in detail explained further in the description of the current invention.

### Technical problem solved by the invention

"The probe for measurement of the time of water penetration through the soil layers and the vertical soil moisture profile" uses FDR - capacitive method to determine the time of water penetration through the soil layers and the vertical soil moisture profile simultaneously (in one cycle of measurement) and includes all soil layers from the soil surface to the plant's root. Compared to the available probes, the probe according to the embodiment disclosed is autonomous wireless device with own battery power which last 3 to 5 years, and does not require any additional auxiliary equipment. The probe has a built-in microprocessor module and radio transmitter for wireless communication and networking with a local server, or to a cloud hosting centre via an Internet connection.

In addition to the time of water penetration through the soil and the soil moisture of multiple layers simultaneously, the probe also measures the parameters from the plant's growing environment, including: temperature at the soil surface and at the root area, then temperature, pressure and humidity of the air and the intensity of the ambient lighting.

The probe also features a NFC (Near Field Communication) module, which enables automatic bi-directional transfer of data to a mobile device (smartphone or tablet), only by bringing the mobile device close to the probe, at a distance of 5- 10 cm.

Usually, the probe sends the data from the sensors to the server database, where it is further processed while the mobile (smartphone, tablet) or a web (desktop, notebook) client application, enables the user to have a detailed overview of measured data coming from all active probes present at all irrigation zones.

The probe is usually placed near the plant, parallel to its stem, while its length should be sufficient to allow measurement up to the root level.

Measuring the time for water penetration through the soil layers and soil moisture in multiple layers simultaneously, is achieved by usage of multiple moisture sensors mounted vertically on top of each other inside of a tube made of plastic or fibreglass material. Usually, the number of built-in sensors is 3-10, and depends on the length of the probe, i.e. the required resolution expressed by the height of the virtual soil layers included in the measurement. Thus, if the probe has 6 sensors and its length is 30 cm, then the resolution (height of each virtual soil layer covered by a sensor) would be 5 cm.

The changes of the moisture in the soil layers are usually slow. However, during irrigation or raining, depending on the amount and duration of the water inflow, it can be registered rapid changes in the soil moisture in a very short interval of time, often expressed in minutes or tens of seconds. Since the flow of the water is usually from the surface, the properties of the soil (structure, composition etc.) may additionally influence the time of water penetration to the root.

The probe abstracts this problem in the way that, the sensors measure the penetration time of the water per layer in real-time, from one layer to another, informing us about the exact depth location (layer in the soil), till which the water has been penetrated.

This gives us the opportunity to timely stop the water supply, and avoid excess water in the irrigated zone, regardless of the soil structure (sand, clay, soft, or hard soil).

Also, the relative moisture of the soil layers by the depth can be quite different. Namely, the water in the upper layers evaporates more rapidly, especially at higher air temperatures and in presence of wind, while in the deeper layers around the root it generally decreases due to the absorption by the plant it needs for its development, but also due to further penetration to greater depths and lateral around the root.

The time of penetration of the water through the soil (seconds / layer), layer by layer from the surface to the root, is determined by comparing the moisture change in each soil layer separately, measured by appropriate sensor incorporated in the probe.

Thus, we get the data for:
1) Time elapsed for water to penetrate through each layer separately;
2) Soil moisture for each layer separately;
3) Current depth, i.e. the layer to which the water penetrated, moving towards the root;
4) Direction of movement of water which is usually from the surface towards the root. However, in the event of emergence of water from sources not under our control, the probe will detect its presence as an irregularity and determine the direction it comes from, laterally or from the area under the probe.

The key parameters that the probe generates (the time of water penetration through the soil layers and the moisture of the soil layers), enable the implementation of a highly accurate irrigation system for optimal supply of water and fertilizer for growing agriculture crops.

The measured soil moisture data is needed for maintaining it at optimum level according to the crop's profile, while the time of water penetration through the layers, for precise control of the irrigation system, i.e. preventing water and fertilizer leakage, monitoring of uncontrolled water inflow from other sources etc.

When the probe for measurement of the time of water penetration through the soil layers as well as the vertical soil moisture profile, is incorporated as a key component in an irrigation system for supply of water and fertilizer, it solves several key problems usually present in the agricultural production, such as:
a) Problem: Inaccurate water supply and liquid fertilizer during irrigation of crops, which can lead to increased soil moisture, which in turn causes the development of fungi, harmful insects and plant diseases; Unnecessary spillage of water and fertilizer above the crop needs, increases the production costs for water, energy and labour, but it also causes dilution and outflow of fertilizer to the soil, or from the fertilizer substrate for greenhouse production.
   Solution: The probe, subject of this invention, enables supply of optimum amounts of water and fertilizer, and prevents irregularities that occur in case of their leakage.
b) Problem: Usage of Plant Growth Regulation (PGR) chemicals. Usually, the increased moisture in the soil is not appropriate for the needs of the agriculture crops and leads to uncontrolled growth and other disturbances. As a consequence, the crop develops an increased leaf mass and decrease in the development of crop's fruits, both in their number and mass. To control the crop growth, producers often use plant growth regulation chemicals, which significantly affect the quality of the products. In addition, usage of PGR chemicals in organic production is not permitted. Solution: By incorporation of the probe, subject to this invention, into an irrigation system with possibility for decision making based on real-time measured data from the soil and the environment, including predefined crop's profile data (plant's needs of water and fertilizer throughout its overall lifecycle), than it will provide much accurate supply of water and fertilizer required for optimal development at every stage of the crop's lifecycle.
c) Problem: Impact of the agricultural production on the environment (increased supply of water and fertilizer above the needs not only increases production costs, but also increases the consumption of additional natural resources for energy production, fuel, transport and storage. Solution: The probe, subject to this invention, provides optimum water supply and fertilizer according to the needs of the crop, thus actively contributes to the conservation of the natural resources. Additionally, by controlling the development of diseases, fungi and pests, the probe also contribute to the preservation of the environment.

### Detailed description of the invention

The probe for measurement of the time of water penetration through the soil layers as well as the vertical soil moisture profile uses Frequency Domain Reflectometry (FDR) technology, more specifically a capacitive method to measure the volume of water present in the soil, expressed in percents [%]. The probe of the invention is defined in claim 1.

Typically, the soil consists of 55% solids (peat, sand, pebbles), air and water. Totally dry soil contains 55% solids and 45% air.

By penetrating into the soil, the water fills in the free air space between the solid particles and increases own volume at the expense of the air. The maximum volume (volumetric content) that water can reach is 45% when we say that the soil is completely saturated with water, i.e. the relative moisture of the soil is 100%.

The dielectric constant of the dry matter in the soil is 3 to 4, for the air it is 1, while for the water it is 80. So, the volumetric content of the water has a dominant influence on the total dielectric constant of the soil, since the amount of solid matter is constant, and the influence of the present air is negligible. By changing the volumetric water content in the soil, its dielectric constant changes proportionally.

The sensors, which are embedded along the soil moisture probe, are actually capacitors whose electrodes are positioned so that the soil plays a role of a dielectric between them. More precisely, the electric field along of each sensor extends horizontally around the probe, in the soil layer covered by the sensor. So, the soil moisture sensors measure the capacity between the electrodes alocated along the inside of the plastic tube inserted into the soil.

Sealed inside in a waterproof tube, the sensors are well protected and completely resistant to the influence from the environment (water, fertilizers and any other chemicals used in the Agriculture.

Primarily, the probe is designed to measure the time of water penetration through the soil layers and the vertical soil moisture profile. However, the probe also measures other parameters from the crop's environment, indoors or outdoors under rigorous operating conditions, from very low to very high temperatures, at dry weather but also at rain and snow.

For detailed explanation of the probe for measurement of the water penetration time through the soil layers and the vertical soil moisture profile, we will use following images:
- Figure 1: Basic outlook of the probe
- Figure 2: Detailed view of the probe's functional modules

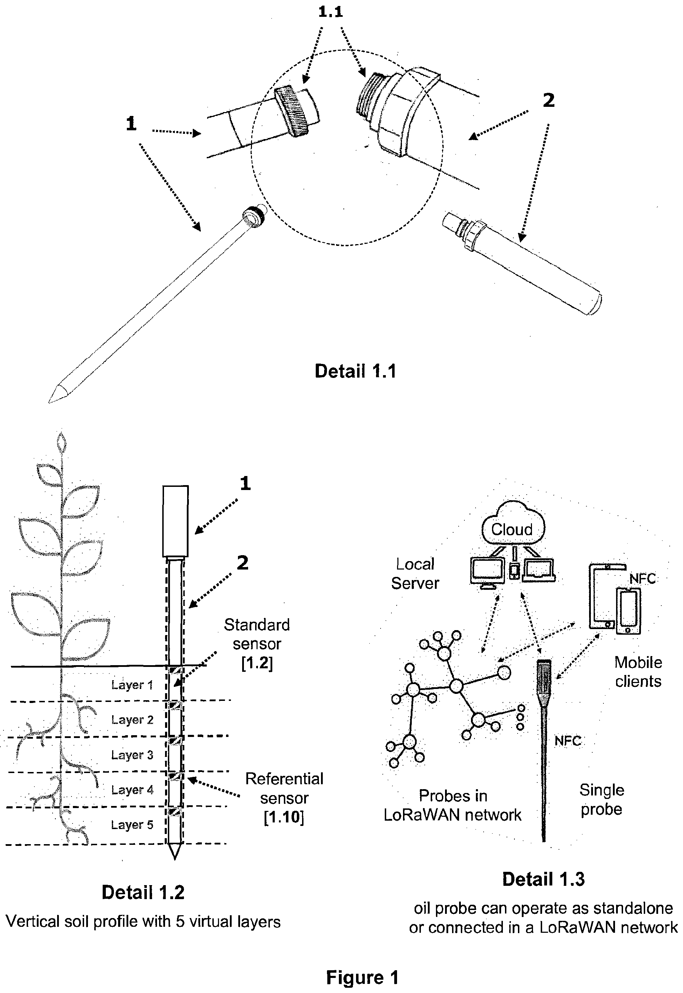

The probe consists of two main parts (Fig. 1, Detail 1.1), interconnected to each other by a waterproof connector [1.1] that tightly couples them into a single device. This connector, in addition to mechanically connecting the two parts, provides their electrical connection too. Both parts are waterproof, and made of hard plastic resistant to wear and cracking at low and high temperatures.

The bottom part [1] is a plastic pipe with diameter of 12-18 mm, where the soil moisture sensors are incorporated. To function properly, the probe requires at least 3 sensors, one located in the layer at the soil surface, the third one in the root region, and the second one between them. Measurements of the time of penetration of water through the soil and the moisture in its layers between the surface and the root, could be provided with higher resolution and accuracy in case the probe has more than 3 sensors installed, enabling in that case more precise control of the supply of water and fertilizer to the crops during the irrigation process.

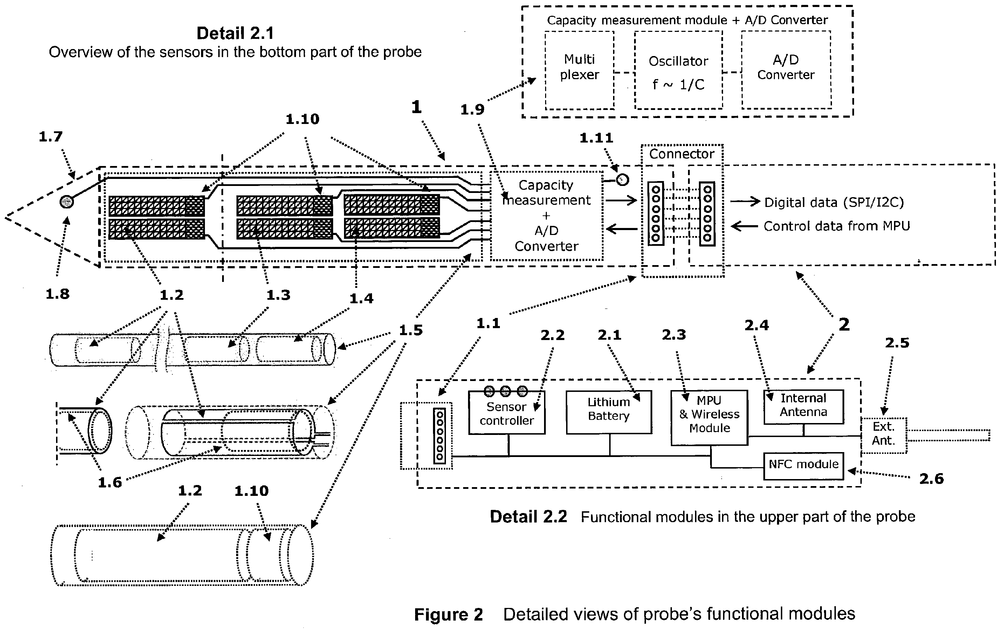

The upper part [2] is a cylindrical plastic case with 30-40 mm diameter and incorporates the following electronic components (Figure 2, Detail 2.2):
- Microprocessor Module (MPU) [2.3], 32Bit, with built-in wireless radio transmitter with frequencies (2.4GHz, 868MHz, 433MHz) which can be freely used in most countries worldwide, and don't need any permission from the authorities. The wireless module uses LoRaWAN communication protocol which provides greater range at lower power consumption than other standards.
- Controller with built-in sensors [2.2] for measurement of the temperature at the soil surface and at the root area, then temperature, humidity and pressure of the ambient air, as well as the intensity of the ambient lighting. Each of these sensors has its own I/O address and outputs a digital data (SIP, I2C) that are directly processed by the MPU module [2.3];
- NFC module [2.6] for wireless data exchange with a mobile device at a distance of 5-10 cm;
- Indoor antenna [2.4], enables range of up to 300m, and an outdoor antenna [2.5] with a range of over 1,000 m, that can be connected if needed;
- Battery [2.1], to power all probe modules including sensors located at the bottom part of the probe [1]. Battery life is 3-5 years, ensured by specially developed software for optimum power supply of each module built into the probe.
- The male part of the connector [1.1] for connecting to the bottom part [1] of the probe which incorporates the sensors for measurement of the time of water penetration through the soil and soil moisture in multiple layers, as well as the sensors for measurement of the temperature at the soil surface and at the root area.

The bottom part [1] of the probe (Figure 2, Detail 2.1) houses:
- Female part of the connector [1.1] for connection to the upper part [2] of the probe,
- Sensors for measurement of the time of penetration of water through the soil and the soil moisture in the layers from the surface to the root area (Fig.2 [1.2], [1.3], [1.4], etc.), They are located at different depths, where they are forming virtual layers of soil which are subject of measurement of the soil moisture within each layer separately (Fig. 1, Detail 1.2; Layer 1 - Layer 5). Their number depends on the length of the probe and the required resolution (height) of the virtual soil layers;
- Sensor for measurement of the temperature at the soil surface [1.11], and
- Sensor for measurement of the temperature in the root area [1.8], located at the metal tip of the probe [1.7].

The probe uses an indirect method to measure the volumetric amount of water in the soil by determining the dialectic constant of the soil. Each sensor consists of two copper plates mounted on a nonconductive waterproof and flexible foil [1.5].

Each pair of copper foil plates forms a capacitor with electric lines applied to the same flexible foil, in the form of a printed circuit board. If the probe measures soil moisture in 5 layers at a time, then the foil will have applied 5 pairs of copper plates with separate electrical lines (flat wires). The foil [1.5] is inserted into the plastic tube [1] by bending it in a form of cylinder with diameter smaller than that of the plastic tube.

This way, the sensor plates form a capacitor with cylindrical surface whose electric field extends directly across the soil, so that the soil becomes a dielectric material between the plates. The cylindrical shape of the electrodes is needed to increase the sensitivity of the sensor, i.e. to increase the surface area of its plates. The sensor with cylindrical plates has about 50% increased effective surface area compared to a flat surface sensor where the shorter side of the electrodes are equal to the diameter of the tube (Perimeter of a circle: C = D * π).

Additionally, in order to direct the electric field from the sensor plates to the soil, but also to reduce its harmful impact through the inner side of the tube on the other sensors, a protective metal shield (copper foil) is inserted under the sensor plates in the cylinder tube [1.6] shown in Fig. 2, Detail 2.1, which is connected to the ground (to the negative pole of the power supply).

By changing the volume of water in the soil, its dielectric constant also changes, resulting in a change in the value of the capacity measured by the sensor. The sensor is included in an L-C oscillator circuit which is part of the module for capacity measurement and A/D conversion [1.9]. So, the oscillator's frequency depends on sensor's capacity (f ~ 1/C), i.e. the soil moisture.

Measured capacity of each sensor is then converted to a digital data (SIP, I2C) which ensures direct communication with the MPU module and easy further processing.

The module for capacity measurement and A/D conversion [1.9] also contains a multiplexer of signals where the number of channels is equal to the number of built-in sensors. Its role is to connect sequentially the sensors one at a time in the oscillator circuit, ensuring measurement of the capacity of each sensor separately and its conversion into digital form.

The whole scanning process is very fast, so that over 100 cycles of complete vertical soil moisture profiles are measured in one second, which comprises the measured moisture values of all soil layers covered by the sensors embedded in the probe.

The accuracy of the soil moisture measurement probe is 3-5% and the repeatability of the measured data is 2%; it can be increased by further correction of the measured data by including correction parameters that affect the accuracy, such as soil temperature, soil composition and structure etc. In addition, by digitizing the measured data immediate at the sensors area, the impact from the environment to the measurement accuracy is significantly reduced.

However, to significantly improve the accuracy of the probe, it is necessary to eliminate the errors from the environment; especially those caused by the influence of the soil temperature changes, the soil structure and composition etc. In that regards, the probe uses a comparative method of measurement by introduction of an additional, referential sensor [1.10] (Fig. 2, Detail 2.1). So, the moisture value for each layer of soil from the vertical profile is calculated as follows: $SMc = kr * \left(MScm*ke-MSzm*ke\right) / \left(MRcm*ke-MRzm*ke\right)$
SMc = Calculated (corrected) soil moisture
kr = Coefficient of adjustment of presented data in "%" of relative moisture
ke = Coefficient of Impact from the soil environment (influence related to the soil temperature changes, soil structure, composition, etc.)
MScm = Measured Moisture of Standard Sensor at current moisture
MSzm = Measured Moisture of Standard Sensor at zero moisture
MRcm = Measured Moisture of Referential Sensor at current moisture
MRzm = Measured Moisture of Referential Sensor at zero moisture

The measured data of moisture at zero volume of water, generated by the standard and the referential sensors, has negligible values and can be considered as zero, so the formula would be: $SMc = kr * \left(MScm*ke\right) / \left(MRcm*ke\right) = kr * \left(ke/ke\right) * \left(MScm\right) / \left(MRcm\right) = kr * MScm / MRcm$

The "ke" coefficient, which here represents the influence of the changes in the soil environment (soil temperature, soil structure and composition, etc.), actually equally affects the measurement accuracy of the standard and the referential sensors. In that regards, the "ke" coefficient in the formula truncates, so its effect on accuracy is reduced to zero.

Compared to the standard measurement, in this case, after each measurement of the soil moisture with a standard sensor, a second measurement follows done by a referential sensor, while the final moisture value is automatically calculated by the MPU module, according to the specified formula. In this case, the accuracy of the probe using a referential sensor is significantly improved (<1%).

In order to achieve the required accuracy, the length of the referential sensor is 15-20% of the length of the standard one (Fig. 2, Detail 2.1). Each referential sensor is fitted above the standard one (closer to the soil surface) so that it always makes the first measurement and the standard sensor the second one. The addition of a separate referential sensor to each standard one is required to address any possible differences between the soil layers (different temperature changes, composition and structure in each separate layer).

Of course, this method could also be accomplished by using just one referential sensor placed next to the first sensor (at the surface layer), next to the last sensor (at the root layer), or somewhere between them. Although in this case - which does not form part of the invention as claimed - we also get increased accuracy, it should be kept in mind that this is a compromised solution (as accurate measurement could be considered only the one for the layer where a referential sensor has been placed).

### Application of the invention

In general, the probe supports several modes of operation (Fig. 1, Detail 1.3):
a) Standalone mode of operation with a mobile device, whereby the measured data is stored in the mobile device (smartphone, tablet) and could be presented in spreadsheet and graphical format,
b) Working in a LoRaWAN network, whereby multiple probes via gateway device (up to 300 probes supported by one gateway) are connected to a local server or to a cloud through an internet connection, and
c) Combination of both modes of work In general, the main application of the probe is in the irrigation systems for optimal supply of water and fertilizers according to the plant's profile, i.e. plant's needs throughout its overall lifecycle. The probe is an essential tool that helps the agriculture producers in their daily activities. It enables precise measurement and storing of the soil and environmental parameters in a knowledge database, which the irrigation system uses for optimal and smart supply of the crops with water and fertilizers according their current needs. Also, it is used for making business decisions during the processes of production planning and management, as well as for business process automation and monitoring of the overall production.

The probe could be used also as a core component in the modern systems for automatic management of agricultural production, i.e. agriculture Decision Support System (DSS), where the system makes decisions based on prior knowledge (knowledge database with data from the growing environment), real time measured data, and the crop's profile data. The optimal needs of one crop are defined in its profile, which includes the optimal moisture and fertilizer required throughout its overall lifecycle. As the needs of the crops change during its development, the irrigation system performs optimal water and fertilizer deliveries exactly according to the data set in its profile, exactly as it is specified for a current period of time.

The maximum benefits of the probe could be obtained when it is used to control a single irrigation zone. The irrigation zone is defined as an arable area planted with a same culture (same profile needs), and the soil has similar characteristics throughout the whole zone (soil structure and composition, ambient lighting, etc.). If for example, there are differences of the soil properties within the same irrigation zone, than that will lead to a different time requirements for water penetration to the root. In such case, the zone should be split in separate areas with similar irrigation characteristics and redefined as new irrigation zones. Otherwise, instead of optimal irrigation, within the irrigation zone some crop areas will be supplied with optimum amount of water and fertilizer, while others with less or more than optimal.

The probe could be also used for automation of agricultural production. Namely, the NFC module built into the probe enables direct data exchange (reading/writing) with NFC enabled mobile devices (smartphones, tablets).

For example, the user could visually locate a probe in the field, in an area where he has observed irregularities in crop's development, and by simply touching the mobile device to the probe, will obtain data on all activities that have taken place during last few days or weeks in the zone controlled by the probe.

Additionally, using the knowledge database and ERP software solution for agricultural production planning and management, the probe's NFC feature can be used for automation of the procedures for quality assurance and control, like in the example:
a) Through a mobile application, the employee receives a request to perform an assignment in a certain area with planted crops;
b) The employee approaches the required zone and using the mobile device, touches the probe that controls the zone. With this action, the employee announces that has arrived at the designated location and begins with the work task;
c) The employee repeats the same action once finishes the work assignment, confirming that has completed the task in the assigned zone within a certain period of time.

The ERP-related mobile application could enable input of additional information and parameters related to the work tasks assigned to the employees, and additionally to record all those data in the knowledge database of an Production Monitoring system and the Quality Assurance and Control system.

## Claims

1. A probe for measurement of the time of penetration of water through virtual soil layers and the vertical moisture profile of the soil, consists of two parts interconnected by a waterproof connector [1.1] that tightly connects the two parts into a single device and electrically connects the two parts, made of solid plastic tube, resistant to wear and cracking at low and high temperatures, while the bottom part [1] is a12-18 mm plastic tube housing at least 3 soil moisture sensors ([1.2], [1.3], [1.4]),
and the upper part [2] is cylindrical plastic box with 30-40 mm diameter containing the electronic components: microprocessor (MPU) module with built-in wireless radio transmitter [2.3], operating in the frequency range 2.4GHz, 868MHz and/or 433MHz; sensor controller [2.2] with sensors for measurement of temperature at the soil surface [1.11] and the root layer [1.8],
also sensors for temperature, humidity and pressure of the air, and intensity of the ambient lighting, wherein the soil moisture sensors are mounted inside the plastic tube on top of each other positioned on different depths, so that when inserted into the soil between the surface and the root, with the first sensor positioned at the surface layer and the last sensor positioned at the root layer, the probe allows for measuring simultaneously the soil moisture at all virtual soil layers where the sensors are positioned and at the same time measuring the time for penetration of the water through each virtual soil layer, wherein an additional referential sensor [1.10] is added to each moisture sensor, whose length is 15-20% of the moisture sensor's length, and the MPU module [2.3] and the sensor controller [2.2] are arranged to carry out a comparative measurement method involving respective standard moisture sensor and referential sensor of each layer, in order to eliminate environmental influences such as changes in soil temperature, soil structure and soil composition in each virtual soil layer included in the measurement separately.

2. The probe according to claim 1 is **characterized in that** soil moisture sensors are used, whose plates are cylindrical in shape and mounted inside the cylindrical protective tube, creating a capacitor with cylindrical plates whose electric field extends directly through the soil, which becomes a dielectric material between the plates, while under the foil on which the sensors are mounted, an additional flexible metal shield is inserted into the cylindrical tube [1.6].

3. The probe according to any of claims 1 and 2, **characterized in that** each sensor has its own I/O address and outputs digital data (SIP, I2C) that could be directly processed by the MPU module [2.3], while other nearby supporting modules are: the sensor controller [2.2], an internal antenna for short range communication < 300m[2.4], an external antenna for extended range communication > 1,000 m [2.5], and lithium battery [2.1] which powers all modules in the probe, including the moisture sensors located in the lower part [1] of the probe.

4. The probe according to any of claims 1 and 2, is **characterized in that** the sensors for measurement of the soil moisture ( [1.2], [1.3], [1.4]) are located at different depths when the probe is inserted in the soil, thereby forming said virtual layers of soil, and the MPU module (2.3) and the sensor controller (2.2) are arranged to carry out cyclic measurements of the time of water penetration through the soil, by comparison of the changes in moisture in each of the virtual soil layers covered by respective sensors at the virtual layers at predetermined depths.

5. The probe according to any of claims 1 to 4, the probe is **characterized in that** the soil moisture sensor is part of an L-C oscillatory circuit whose frequency is changed by changing its capacity related to the soil moisture content, which is further processed in an A/D module that outputs data in digital format (SIP, I2C), which is further processed by the MPU module.

6. The probe according to any of the preceding claims, wherein an embedded NFC module [2.6] enables bi-directional data exchange at distance of 5-10 cm by using a mobile device such as a smartphone or tablet.

## Patentansprüche

1. Sonde zur Messung der Eindringzeit von Wasser durch virtuelle Bodenschichten und des vertikalen Feuchtigkeitsprofils des Bodens, bestehend aus zwei Teilen, die durch einen wasserdichten Verbinder [1.1] miteinander verbunden sind, welcher die beiden Teile dicht zu einem einzigen Gerät verbindet und sie elektrisch miteinander verbindet, hergestellt aus einem massiven Kunststoffrohr, das verschleiß- und rissbeständig bei niedrigen und hohen Temperaturen ist, wobei der untere Teil [1] ein 12-18 mm Kunststoffrohr ist, das mindestens 3 Bodenfeuchtigkeitssensoren aufnimmt, und der obere Teil [2] ein zylindrisches Kunststoffgehäuse mit einem Durchmesser von 30-40 mm ist, das die elektronischen Komponenten enthält: ein Mikroprozessormodul (MPU) mit eingebautem drahtlosem Funksender [2.3], der im Frequenzbereich 2,4 GHz, 868 MHz und/oder 433 MHz arbeitet; einen Sensorcontroller [2.2] mit Sensoren zur Messung der Temperatur an der Bodenoberfläche und in der Wurzelschicht [1.8], sowie Sensoren für Temperatur, Luftfeuchtigkeit und Luftdruck sowie die Intensität des Umgebungslichts, wobei die Bodenfeuchtigkeitssensoren innerhalb des Kunststoffrohrs übereinander in unterschiedlichen Tiefen angeordnet sind, sodass beim Einführen in den Boden zwischen der Oberfläche und der Wurzel, wobei der erste Sensor an der Oberflächenschicht und der letzte Sensor an der Wurzelschicht positioniert ist, die Sonde es ermöglicht, gleichzeitig die Bodenfeuchtigkeit in allen virtuellen Bodenschichten, in denen die Sensoren positioniert sind, zu messen und gleichzeitig die Eindringzeit des Wassers durch jede virtuelle Bodenschicht zu messen, wobei jedem Feuchtigkeitssensor ein zusätzlicher Referenzsensor [1.10] zugeordnet ist, dessen Länge 15-20 % der Länge des Feuchtigkeitssensors beträgt, und das MPU-Modul [2.3] und der Sensorcontroller [2.2] dazu eingerichtet sind, ein Vergleichsmessverfahren unter Einbeziehung des jeweiligen Standard-Feuchtigkeitssensors und des Referenzsensors jeder Schicht durchzuführen, um Umwelteinflüsse wie Änderungen der Bodentemperatur, der Bodenstruktur und der Bodenzusammensetzung in jeder einzeln in die Messung einbezogenen virtuellen Bodenschicht auszuschließen.

2. Sonde nach Anspruch 1, **dadurch gekennzeichnet, dass** Bodenfeuchtigkeitssensoren verwendet werden, deren Platten zylindrisch geformt und innerhalb des zylindrischen Schutzrohrs montiert sind, wodurch ein Kondensator mit zylindrischen Platten entsteht, dessen elektrisches Feld sich direkt durch den Boden erstreckt, welcher zu einem dielektrischen Material zwischen den Platten wird, wobei unter der Folie, auf der die Sensoren montiert sind, eine zusätzliche flexible Metallabschirmung in das zylindrische Rohr [1.6] eingesetzt ist.

3. Sonde nach einem der Ansprüche 1 und 2, **dadurch gekennzeichnet, dass** jeder Sensor seine eigene Ein-/Ausgabeadresse besitzt und digitale Daten (SIP, I2C) ausgibt, die direkt vom MPU-Modul [2.3] verarbeitet werden können, wobei die weiteren in der Nähe befindlichen Hilfsmodule sind: der Sensorcontroller [2.2], eine interne Antenne für Kurzstreckenkommunikation < 300 m [2.4], eine externe Antenne für Kommunikation über größere Reichweite > 1.000 m [2.5], sowie eine Lithiumbatterie [2.1], die alle Module der Sonde einschließlich der im unteren Teil [1] der Sonde befindlichen Feuchtigkeitssensoren mit Strom versorgt.

4. Sonde nach einem der Ansprüche 1 und 2, **dadurch gekennzeichnet, dass** die Sensoren zur Messung der Bodenfeuchtigkeit ([1.2], [1.3], [1.4]) beim Einführen der Sonde in den Boden in unterschiedlichen Tiefen angeordnet sind und dadurch virtuelle Bodenschichten bilden, und das MPU-Modul [2.3] und der Sensorcontroller [2.2] dazu eingerichtet sind, zyklische Messungen der Wassereindringzeit durch den Boden durchzuführen, indem die Feuchtigkeitsänderungen in jeder der virtuellen Bodenschichten verglichen werden, die von den jeweiligen Sensoren an den virtuellen Schichten in vorbestimmten Tiefen erfasst werden.

5. Sonde nach einem der Ansprüche 1 bis 4, **dadurch gekennzeichnet, dass** der Bodenfeuchtigkeitssensor Teil eines LC-Schwingkreises ist, dessen Frequenz durch Änderung seiner Kapazität in Abhängigkeit vom Bodenfeuchtigkeitsgehalt verändert wird, welche anschließend in einem A/D-Modul verarbeitet wird, das Daten im digitalen Format (SIP, I2C) ausgibt, die anschließend leicht vom MPU-Modul verarbeitet werden können.

6. Sonde nach einem der vorhergehenden Ansprüche, wobei ein eingebettetes NFC-Modul [2.6] einen bidirektionalen Datenaustausch in einem Abstand von 5-10 cm mittels eines mobilen Geräts wie eines Smartphones oder Tablets ermöglicht.

## Revendications

1. Sonde de mesure du temps de pénétration de l'eau à travers des couches virtuelles de sol et du profil vertical d'humidité du sol, composée de deux parties reliées entre elles par un connecteur étanche [1.1] qui assure la connexion étanche des deux parties en un seul dispositif et assure leur connexion électrique, réalisée en tube de plastique massif, résistant à l'usure et à la fissuration à basses et hautes températures, la partie inférieure [1] étant un tube en plastique de 12 à 18 mm logeant au moins 3 capteurs d'humidité du sol, et la partie supérieure [2] étant un boîtier cylindrique en plastique d'un diamètre de 30 à 40 mm contenant les composants électroniques : un module microprocesseur (MPU) doté d'un émetteur radio sans fil intégré [2.3], fonctionnant dans la plage de fréquences 2,4 GHz, 868 MHz et/ou 433 MHz ; un contrôleur de capteurs [2.2] muni de capteurs pour la mesure de la température à la surface du sol et au niveau de la couche racinaire [1.8], ainsi que des capteurs de température, d'humidité et de pression de l'air, et d'intensité de la lumière ambiante, dans laquelle les capteurs d'humidité du sol sont montés à l'intérieur du tube en plastique, superposés les uns au-dessus des autres à différentes profondeurs, de sorte que, lors de l'insertion dans le sol entre la surface et la racine, le premier capteur étant positionné au niveau de la couche superficielle et le dernier capteur au niveau de la couche racinaire, la sonde permette de mesurer simultanément l'humidité du sol dans toutes les couches virtuelles de sol où les capteurs sont positionnés et de mesurer en même temps le temps de pénétration de l'eau à travers chaque couche virtuelle de sol, dans laquelle un capteur référentiel supplémentaire [1.10] est ajouté à chaque capteur d'humidité, dont la longueur représente 15 à 20 % de la longueur du capteur d'humidité, et le module MPU [2.3] et le contrôleur de capteurs [2.2] sont agencés pour exécuter une méthode de mesure comparative impliquant respectivement le capteur d'humidité standard et le capteur référentiel de chaque couche, afin d'éliminer les influences environnementales telles que les variations de température du sol, de structure du sol et de composition du sol dans chaque couche virtuelle de sol prise en compte séparément dans la mesure.

2. Sonde selon la revendication 1, **caractérisée en ce que** les capteurs d'humidité du sol utilisés ont des plaques de forme cylindrique montées à l'intérieur du tube de protection cylindrique, formant un condensateur à plaques cylindriques dont le champ électrique s'étend directement à travers le sol, lequel devient un matériau diélectrique entre les plaques, tandis que, sous le film sur lequel les capteurs sont montés, un blindage métallique flexible supplémentaire est inséré dans le tube cylindrique [1.6].

3. Sonde selon l'une quelconque des revendications 1 et 2, **caractérisée en ce que** chaque capteur possède sa propre adresse d'entrée/sortie et fournit des données numériques (SIP, I2C) pouvant être directement traitées par le module MPU [2.3], les autres modules auxiliaires à proximité étant : le contrôleur de capteurs [2.2], une antenne interne pour communication à courte portée < 300 m [2.4], une antenne externe pour communication à portée étendue > 1 000 m [2.5], et une batterie au lithium [2.1] alimentant tous les modules de la sonde, y compris les capteurs d'humidité situés dans la partie inférieure [1] de la sonde.

4. Sonde selon l'une quelconque des revendications 1 et 2, **caractérisée en ce que** les capteurs de mesure de l'humidité du sol ([1.2], [1.3], [1.4]) sont situés à différentes profondeurs lorsque la sonde est insérée dans le sol, formant ainsi des couches virtuelles de sol, et le module MPU [2.3] et le contrôleur de capteurs [2.2] sont agencés pour effectuer des mesures cycliques du temps de pénétration de l'eau à travers le sol, par comparaison des variations d'humidité dans chacune des couches virtuelles de sol couvertes par les capteurs respectifs situés aux couches virtuelles à des profondeurs prédéterminées.

5. Sonde selon l'une quelconque des revendications 1 à 4, **caractérisée en ce que** le capteur d'humidité du sol fait partie d'un circuit oscillant L-C dont la fréquence est modifiée par la variation de sa capacité en fonction de la teneur en humidité du sol, laquelle est ensuite traitée dans un module A/N qui délivre des données au format numérique (SIP, I2C), lesquelles sont ensuite aisément traitées par le module MPU.

6. Sonde selon l'une quelconque des revendications précédentes, dans laquelle un module NFC intégré [2.6] permet un échange de données bidirectionnel à une distance de 5 à 10 cm au moyen d'un dispositif mobile tel qu'un smartphone ou une tablette.
